# EUROPEAN PATENT SPECIFICATION

(11) **EP 0 986 404 B1**
(45) Date of publication and mention of the grant of the patent: **17.04.2002**
(21) Application number: 97930670.1
(22) Date of filing: 10.07.1997
(51) Int. Cl.: A61K 47/48, A61K 9/51

(54) **GENE THERAPY DELIVERY SYSTEM FOR TARGETING TO ENDOTHELIA**
GENTHERAPIESYSTEM ZUR ZIELUNG DES ENDOTHELIUMS
SYSTEME D'ADMINISTRATION D'UNE THERAPIE GENIQUE CIBLEE SUR L'ENDOTHELIUM

(30) Priority: 10.07.1996 GB 9614441; 10.07.1996 GB 9614471
(43) Date of publication of application: 22.03.2000
(73) Proprietor: West Pharmaceutical Services Drug Delivery & Clinical Research Centre Limited, Nottingham NG7 2TN (GB)
(72) Inventor: ILLUM, Lisbeth, Nottingham NG7 1BA (GB)
(74) Representative: McNeeney, Stephen Phillip
(86) International application number: GB9701860
(87) International publication number: WO9801161

(56) References cited:
- WO-A-96/13253
- WO-A-96/20698
- FR-A- 2 724 935
- ALLEMANN E ET AL: "DRUG-LOADED NANAOPARTICLES PREPARATION METHODS AND DRUG TARGETING ISSUES" EUROPEAN JOURNAL OF PHARMACEUTICS AND BIOPHARMACEUTICS, vol. 39, no. 5, 1 October 1993, pages 173-191, XP000403429
- FELGNER P L ET AL: "LIPOFECTON: A HIGHLY EFFICIENT, LIPID-MEDIATED DNA-TRANSFECTION PROCEDURE" PROCEEDINGS OF THE NATIONAL ACADEMY OF SCIENCES OF USA, vol. 84, no. 21, November 1987, pages 7413-7417, XP000602252 cited in the application
- CREMERS H F M ET AL: "ALBUMIN-HEPARIN MICROSPHERES AS CARRIERS FOR CYTOSTATIC AGENTS" JOURNAL OF CONTROLLED RELEASE, vol. 12, no. 1 - 03, 1 January 1990, pages 167-179, XP000113382 cited in the application

## Description

The present invention relates to a novel embolism system for the delivery of gene therapy.

Gene therapy offers exciting opportunities for the treatment of various diseases, including those that are inherited and conditions such as cancer and asthma, as well as for immunization (DNA vaccines). An important aspect in the success of gene therapy will be the development of appropriate delivery systems (often termed "vectors") that provide the gene construct with access to the target organ and, more particularly, to the target cells wherein transfection will occur. A good example is the treatment of inherited and acquired diseases in the lung (Curiel *et al.* (1996) *Am. J. Resp. Cell. Mol. Biol*. **14,** 1-18).

Two drug delivery routes are available for lung treatments. The drug can be delivered via the mouth in the form of an aerosol into the lung airways. For example, the preparation of lipid carrier-nucleic acid complexes for delivery to the lung airways by aerosol are described in PCT WO93/1275. Such delivery is known to be extremely inefficient using existing powder and aerosol devices. More efficient delivery can be achieved using various nebulizer systems but these devices can cause degradation of the gene construct and also require substantial periods of time for a dose to be administered. Moreover, in many clinical conditions such as cystic fibrosis, the lungs contain mucus that prevents deep penetration of the delivery system into the lung and appropriate presentation of the gene construct to the target epithelial cells.

An alternative means of delivery to the lung is to exploit the circulation system and to deliver the gene product to the lung endothelia. The gene product can be injected into the venous or arterial supply and directed to the endothelial layer using appropriate delivery technologies. The prior art describes how this can be achieved using some form of ligand mediated approach using sugars or monoclonal antibodies or their fragments (see for example the studies of Huang, Holmberg *et al*, (1990) *J. Liposome Res*., **1,** 393. This approach, while successful in animal models, is complex and does not lend itself easily to scale-up and manufacture under normal pharmaceutical processing. An alternative approach to delivery to the lung is to use particle systems wherein the particles have a size that will result in the temporary blockage or embolism of the capillary beds of the lungs. Particles larger than 4-5 µm in diameter will be filtered out by the lung capillaries, with high extraction and primarily to the alveolar segments (see for example Kanke *et al*, (1980) *J.Pharm. Sci*. **69,** 755). The human lung is believed to have about 2.8 x 10⁸ alveoli and 2.8 x 10¹¹ capillary segments. Previously, a wide variety of colloidal systems has been examined as lung scanning agents including ¹⁹⁸Au adsorbed onto carbon, ¹¹¹Ag and ²⁰³Hg adsorbed onto ceramic materials, ^{99m}Tc-ferric hydroxide aggregates as well as more recently, macroaggregates and microspheres made from human serum albumin. Metabolisable albumin microspheres labeled with ^{99m}Tc, ¹¹¹In or ^{113m}In are the systems of choice (see Hagan *et al*, (1978) *J. Nucl. Med*., **19,** 1055). Davis has indicated that the size range 13.5 ±1.5 µm diameter would be ideal; however, this was too narrow for commercial production and he has proposed a range 10 to 20 µm (15 ± 5 µm). Such systems have a large margin of safety, provided that the administered dose (particle number) is very small in comparison to the number of capillary segments, the toxicity being related to the size; large particles (90 µm in diameter) are more toxic than smaller ones (Davis, in *Radiopharmaceuticals*, Ed. Subramanian *et al*, Soc. Nucl. Med. New York, 1975, p.267 and Davis and Taube (1978) *J. Nucl. Med*. **19**, 1209). The rate of breakdown of albumin (either in the form of microaggregates or microspheres) is dependent on the so-called "hardness" of the system, which is related to the method of preparation and treatment (heating temperature, cross-linking agents, etc.). Total clearance from the lung field is generally seen within 24 hr but the rate of breakup of the microsphere and the loss of the label do not necessarily occur at the same rate.

Illum *et al* ((1982) *Int. J. Pharm.* **12,** 135 and (1982) *J. Pharm. Pharmacol. Suppl.* **34**, 89P) have used polystyrene particles surface labelled with ¹³¹I and ion exchange (DEAE) cellulose microspheres labelled with ¹³¹I-rose bengal to investigate effects of particle size and particle shape on lung deposition. Polystyrene microspheres of 15.7 µm mean diameter and DEAE-cellulose microspheres 40 to 160 µm diameter at a dose of about 10⁸ particles were rapidly taken up by the lungs and the particles were lodged in the lung capillaries. The lung entrapment and fate of albumin microspheres loaded with the drug adriamycin have been studied by Wilmott *et al* using the rat as an animal model (Wilmott *et al* in *Microspheres and Drug Therapy. Pharmaceutical, Immunological and Medical Aspects* edited by S S Davis, L Illum, J G McVie and E Tomlinson 1984, Elsevier Science Publishers B.V., Amsterdam, p.205). The idea was that the particle would reside in the blocked capillary and thereby allow the retained drug to be released from the particle and diffuse into the tissues and reach the site of action.

A similar embolism concept has been developed by the Swedish company, Pharmacia, with its Spherex (registered trade mark) system. This system is based upon degradable starch microspheres that are administered intravenously or intra arterially so as to reach organ sites in order to create a local embolism before the administration of a pharmacological agent, usually a drug used in the chemotherapy of cancer. The temporary embolism, once again, is intended to hold the drug at the target site for a prolonged period of time in order to maximize absorption of the drug into the surrounding tissues (see Lindberg *et al* in *Microspheres and Drug Therapy. Pharmaceutical, Immunological and Medical Aspects* edited by S S Davis, L Illum, J McVie and E Tomlinson 1984, Elsevier Science Publishers, Amsterdam, p. 153). This embolism system is described for the lung and the liver.

The encapsulation of synthetic oligonucleotides and PCR generated DNA fragments into gelatin microspheres has been described by Cortesi *et al*, (1994) *Int. J. Pharmaceut*. **105**, 181. Neither the attachment of DNA to the surface of preformed microspheres nor the use of such systems for delivery to the endothelial cells in the lung vasculature were described.

H.-Q. Mao, et al. in Proceed. Intern. Symp. Control. Rel. Bioact. Mater., 23 (1996) page 401 and 402 describe a nanosphere delivery vehcile made by the complex of DNA and chitosan.

We have devised a novel embolism system intended for the delivery of genes. Unlike the previously described drug delivery systems for lung targeting, we have deliberately arranged for the gene material to reside on the surface of the biodegradable microsphere that occludes the vascular bed on a temporary basis. In this manner, we have found that the gene product is presented to the endothelial surface in an extremely efficient manner, leading to improved cellular uptake and expression of the gene product. It will be understood that the embolism system may be used for organs other than the lung. For other organs the embolism system may be administered by injecting into the relevant artery.

We have further discovered that it is important that the gene product is itself appropriately formulated, not only to provide good attachment to the surface of the carrier microsphere, but in a form that allows appropriate cell presentation and uptake. We have found that the simple adsorption of uncondensed DNA or plasmid DNA to a microsphere through a process of physical adsorption wherein the microsphere carries a positive charge that allows the strong binding of the negatively charged DNA, does not lead to beneficial expression within a cell culture model. Moreover, it is unlikely that a positively charged microsphere for use as a carrier would be approved for human use since such positively charged microspheres and other particles can be associated with toxic effects upon injection.

In contrast, if the DNA itself is first condensed into small particles of a size less than 1 µm, then good expression of the polypeptide encoded by the DNA can be achieved. Such condensation can be brought about by the use of cationic compacting agents such as cationic lipids or, preferably, by cationic polymers.

A first aspect of the invention is a composition comprising biodegradable microspheres wherein the microspheres carry a net negative charge and to which is adsorbed positively charged particles of a smaller size, wherein such positively charged particles comprise a conjugate of DNA and a cationic compacting agent.

It will be clear to the skilled person that the final composition will carry a net negative charge, will be neutral or will carry a small positive charge, depending on the net charge and size of the microsphere and the surface density and net charge of the adsorbed particles.

A second aspect of the invention is a method of preparing said composition, the method comprising 1) preparation of positively charged particles comprising a conjugate of DNA and a cationic compacting agent, and 2) adsorbing said positively charged particles on to biodegradable microspheres carrying a net negative charge. By "adsorbing" is meant any form of non-covalent binding.

The term "microsphere" is used herein to include both solid matrix microspheres and microcapsules. Biodegradable microspheres that are of the appropriate size and surface charge density can be formulated from a number of different materials, such as soluble starch, carrying carboxyl groups covalently linked to the starch, and polymers that contain groups that ionise to allow electrostatic interaction between positively charged DNA conjugates and the biodegradable microspheres. Such groups are preferably carboxyl or sulphate groups. Such polymeric materials include the alginates, heparin and other sulphated polysaccharides (carboxymethyl dextran, carboxydextran and dextran sulphate) and biodegradable synthetic polymers such as polylactide coglyceride and polylactic acid. Likewise, a physical mixture of polymers that is then converted into microspheres may be used. For example, heparin/albumin microspheres have previously been described for the purposes of drug delivery (Kwon *et al*, (1991) *J. Colloid Interface Sci.* **143,** 501 and Cremers *et al*, (1990) *J. Controlled Release* **11**, 167. Such materials have not been described for use in gene therapy nor for the carriage of DNA conjugates.

Hence, the biodegradable microspheres may be prepared from crosslinked soluble starch, albumin, gelatin, heparin, carboxydextran, dextran, dextran sulphate, alginate, polylactide, or their admixtures.

The biodegradable microspheres, in order to provide embolism, should be preferably have an average size (mean number diameter, as determined by light microscopy) larger than 5 µm, more preferably between 6 and 200 µm, most preferably between 10 and 100 µm and still more preferably between 12-50 µm. The size of the biodegradable microsphere described herein refers to the size of the swollen particle in the vehicle chosen for intravenous administration.

The properties of the carrier system can be so designed as to provide different rates of degradation and thereby control the period of contact between the carrier microsphere and, for example, the lung endothelial cells. The different rates of degradation can be achieved by methods known to those skilled in the art such as regulating the degree of crosslinking of the microspheres; the less crosslinked microspheres degrade faster than the microspheres which are crosslinked to a higher degree. Likewise for some polymers it is possible to control the rate of degradation by selection of lower molecular weight polymer chains for faster degradation and higher molecular weight polymer chains for slower degradation. For systems that are crosslinked by heating, the degree of crosslinking and therefore the rate of degradation can be controlled by the period of heating and the temperature.

In order for the positively charged particles to be adsorbed onto the surface of biodegradable carrier microspheres, the carrier microsphere itself should have a net negative charge (zeta potential) of at least -1 mV, preferably at least -5 mV and more preferably at least -20 mV when measured using microelectrophoresis.

Suitable microparticles for use the compositions of the invention are commercially available and include starch microspheres (such as Cadoxamer (Perstorp) and Spherex (Pharmacia Upjohn)), dextran microspheres (CM Sephadex (Pharmacia Upjohn) and SP Sephadex (Pharmacia Upjohn)) and agarose microspheres (CM Sepharose (Pharmacia Upjohn) and S Sepharose (Pharmacia Upjohn)). Albumin and gelatin microspheres may be prepared according to methods which are well known to those skilled in the art.

Examples of cationic compacting agents include chitosan. By the term "chitosan" we mean polyglucosamine and oligomers of glucosamine materials of different molecular weights and degrees of acetylation. Modified chitosans, particularly those conjugated to polyethylene glycol, are included in this definition. Further examples of cationic compacting agents include, but are not limited to, polyamidoamines (including dendritic derivatives thereof), stearylamine, DOTMA (N-[1-(2,3-dioleoyloxy)propyl]-N,N,N-trimethylammonium chloride), BISHOP (N-[(2,3-dihexadecyloxy)prop-1-yl]-N,N,N-trimethylammonium chloride), DODAC(B) (N,N-dimethyl-N-octadecyl-1-octadeca ammonium chloride (or bromide)), polylysine, lipofectin, lipofectamine, DMRIE (dimyristoyl Rosenthal Inhibitor ether), DC-cholesterol (dimethylaminoethyl carbamyl cholesterol). These compacting agents can be mixed with neutral zwitter ionic lipids such as DOPE (dioleoyl phosphatidyl ethanolamine) in order to elicit optimal effects. Other compacting agents include CTAB (cetyldimethylethyl ammonium bromide), DDAB (dimethyl dioctadecyl ammonium bromide), lipopolyamines such as dioctadecylamido glycylspermine and dipalmitoyl phosphatidyl ethanol amido spermine, cationic derivatives of cholesterol, DOSPA (dioleoyl-N-(2-(sperminecarboxamido)ethyl-N,N-dimethyl-1-propanaminium trifluoroacetate). Such cationic compacting agents can either be used separately or in any combination. Such condensation processes have been described previously in the prior art with particular emphasis on the cationic polymer, polylysine. For example, polyelectrolyte complexes formed between DNA and polylysine have been described by Wolfert and Seymour (1996) *Gene Therapy* **3**, 269.

A preferred aspect of the invention is that the cationic compacting agent is chitosan.

In the present invention, compacted complexes of DNA are produced so that they carry a net positive charge. These are termed "conjugates". This can be accomplished using a cationic compacting agent as described above by titrating the cationic compacting agent into a solution of DNA and then measuring both the size of the resultant particle, but more importantly the surface charge using appropriate techniques such as Laser Doppler Anemometry that provides a measure of the zeta potential.

The following example describes a method of preparing chitosan-DNA complexes. The method comprises mixing the two components together, the rate of mixing, the relative concentrations of the starting solution, the ratios of the two components, pH, ionic strength and temperature being such as to achieve the required particle size and charge. The rate of mixing, the relative concentrations of the starting solution, pH, ionic strength and temperature can be altered as part of a standard experimental design to control particle size. The ratios of the two components can also be used to control size and surface charge. The weight to weight ratio between nucleic acid and chitosan can be between 1:1 and 1:25, preferably between 1:2 and 1:20 and most preferably between 1:5 and 1:6.

The size and size distribution of the complexes may be determined by Photon Correlation Spectroscopy (PCS) using a Malvern 4700 submicron particle analyser system (Malvern Instruments, UK) with a PCS 100 spectrometer. The samples are diluted with distilled water and measured at 25°C at a scattering angle of 90°. The size distribution is characterised by a polydispersity index (PI).

The molecular weight of the chitosan influences the size of the compacted DNA complex, and indeed can be chosen so as to influence the size of the complex. To this end studies can be conducted using ethidium bromide fluorescence as described by Wolfert and Seymour (1996) *Gene Therapy* **3**, 269-273. Ethidium bromide interacts with DNA to provide strong fluorescence. When a complexing cationic polymer is added the ethidium bromide is squeezed out of the complex and the fluorescence reduced. A plot of fluorescence against quantity of added polymer can be used to assess the compaction process (loss of fluorescence is indicative of degree of compaction) and thereby the selection of the appropriate molecular weight of the complexing chitosan species. Fluorescence may be measured using Perkin-Elmer 3000 Fluorescence Spectrometer, for example. The optical emission wavelength is set at 591 nm and optical excitation wavelength is set at 366 nm. Ethidium bromide (EtBr) is added to water in a cuvette (reference) and DNA solution and ethidium bromide to are added to water in a second cuvette (test). The fluorescence of the reference cuvette is set to zero. The fluorescence obtained for the test cuvette is measured. Chitosan samples are then introduced in very small portions to the test cuvette with constant stirring, and the fluorescence reading is taken three times, returning to the zero cell in between each reading. The average of the three is then recorded.

The molecular weight of the chitosan is preferably between 500 Dalton and 500,000 Dalton, more preferably 700 Dalton to 250,000 Dalton and most preferably 1,000 Dalton to 150,000 Dalton.

Chitosans of different low molecular weight can be prepared by enzymatic degradation of chitosan using chitosanase or by the addition of nitrous acid. Both procedures are well known to those skilled in the art and are described in recent publications (Li *et al*, (1995) *Plant Physiol. Biochem*. **33**, 599-603; Allan and Peyron, (1995) *Carbohydrate Research* **277**, 257-272; Damard and Cartier, (1989) *Int. J. Biol. Macromol.* **11**, 297-302).

The positive charge of the DNA conjugate (the zeta potential) should be at least +5mV, preferably at least +10 mV and most preferably at least +20 mV, but preferably less than +100 mV.

The surface charge of the conjugates is expressed as a zeta potential. This is determined at pH 7.4 and 0.001 M ionic strength. The zeta potential is measured by Laser Doppler Anemometry (LDA) using for example a Malvern Zeta Sizer IV (Malvern, UK). Laser Doppler Anemometry involves the detection of scattered laser light from particles which are moving in an applied electric field. The equipment used can for example be a Malvern Zetasiser II (Malvern Instruments).

Electrophoretic mobility measurements are carried out by dispersing the conjugates in 10 ml buffer solution such as phosphate or McIlvaine buffer, from 100 µg - 1 mg, with a constant ionic strength 0.001M. Four readings are taken using a PC4 wide capillary cell at a voltage of 100 volt, electrode spacing of 50 mm and a dielectric constant of 78.54.

Such positively charged conjugates with a size between 10-1000 nm can be separated from the excess complexing agent using procedures familiar to those skilled in the art. It is known that column separation methods are less than ideal for investigations with chitosan and similar cationic polymers. Instead, methods such as field flow fractionation using a cross-flow process (FF Fractionation, Salt Lake City, Utah; Ratanathanawongs and Giddings, (1993) *ACT Symp. Ser.* **521,** 13) or continuous split-flow thin (SPLITT) cell can be employed as described by Levin and Giddings, (1991) *J. Chem. Biotechnol.* **50**, 43; Williams *et al* (1992) *Ind. Eng. Chem. Res.* **31,** 2172. At the research level, filter centrifugation tube systems can be used.

In order for the DNA conjugate to provide good expression of the polypeptide encoded by the DNA, the conjugate should preferably be of a size below 1 µm, more preferably below 500 nm and most preferably below 200 nm. The conjugate preferably has a size of at least 10 nm (hydrodynamic diameter, as measured by photon correlation spectroscopy).

By DNA we mean nucleic acids (including RNA) and oligonucleotides greater than 100 base pairs in length or plasmids or cosmids, coiled or uncoiled.

The DNA may be any DNA that expresses polypeptides and protein drugs that are useful in the treatment of diseases both inherited and acquired that are localised in the lung. These include cystic fibrosis (cystic fibrosis transmembrane regulator), alpha 1-antitrypsin deficiency and lung cancer (genes to induce immunity or those that are directly tumoricidal). Immunity inducing genes include those encoding for MHC (major histocompatibility complex) molecules, co-stimulatory molecules and cytokines. Toxic systems include tumor expressed genes (p53) or enzymes that convert non toxic prodrugs to toxic ones. Herpes thymidine kinase is an example.

Pulmonary inflammation, surfactant deficiency, pulmonary hypertension and malignant mesothelioma are other conditions in which the compositions of the invention may be applied. However, the compositions may also be used for delivery of DNA to the lung where the lung is used as a bioreactor for polypeptides and proteins whose main site of action is elsewhere in the body (for example blood factors XIII, IX). By this is meant that the DNA is expressed in the lung tissue, and the expressed polypeptides are secreted from the cells and enter the circulation, with the effect that the polypeptides are distributed to other parts of the body.

It will be appreciated that the invention may also be used to deliver gene therapy into other body compartments such as joints, nasal cavity, vagina, rectum, eye, gastrointestinal tract, ear or buccal membrane. The compositions may comprise any DNA that expresses polypeptides and protein drugs that are useful in the treatment of disease in these compartments. Examples include antiinfective agents, antiinflammatory agents, prophylactic agents and antigens that will lead to humoral and cell mediated responses i.e. antigenic material intended as a vaccine. The DNA may encode a viral, bacterial or parasite protein and thus be useful in vaccination against diseases such as tetanus, diphtheria, whooping cough and influenza. Gene products include but are not limited to alpha 1-antitrypsin, growth hormone synthase, factor VIII, factor IX, TNF, cytokines, antigen genes (hepatitis B, influenza, cholera), the cystic fibrosis transmembrane conductance regulator CTFR, cancer genes (p53, K-ras, HS-Ek), sucrose-isomaltase, lactase-phlorizin hydrolase and maltase-glucoamylase.

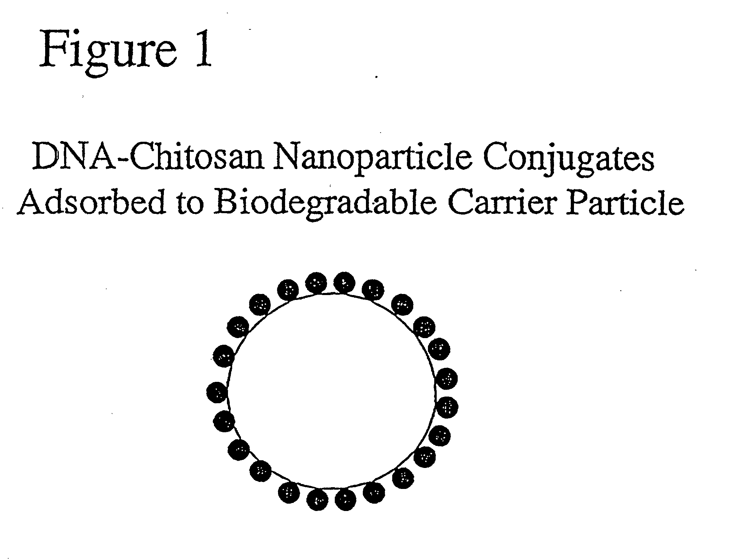

The physical attachment of the conjugate to the larger biodegradable carrier microspheres can be undertaken using conventional formulation methods. The adsorption of the DNA conjugate on the microspheres can be carried out by methods known to those skilled in the art. For example, the microspheres may be suspended in a solvent such as water or a buffer of low ionic strength. The DNA conjugate is added to the suspension and the mixture left at room temperature with gentle shaking for a period of time sufficient to allow adsorption to take place, typically 1 - 3 hours. The excess DNA conjugate is separated from the microspheres by centrifuging at low speed (about 6000 rpm) for about 3 - 5 mins. The process by which one large microsphere carries many small particles on the surface has been described in the colloid science area and is known as "heterocoagulation" (see for example, Buscall *et al, Polymer Colloids,* Elsevier, London, 1985, p. 167). In such coagulation processes, it is important to ensure that the carrier microspheres are uniformly coated with the small nanoparticles rather than resulting in a gel interlinked or bridged system (Figure 1). This may be checked by standard techniques (e.g. microscopy or rheological techniques).

A further aspect of the present invention is the use of the composition of the invention in medicine. It will be important that the dose of the microspheres administered to man or to a mammal is chosen such that only a small percentage of the available organ, for example lung, capillaries are blocked at any one time. The literature in the field of diagnostic imaging provides details of the doses that can be safely administered for such a purpose in diagnostic imaging (see Davis and Taube (1978) *J. Nucl. Med.* **19,** 1209). In practice, not more than 10% of the lung capillaries should be so blocked. A suitable range of microspheres would be less than 10 x 10¹⁰ in number, preferably 10⁶ - 10⁸ and more preferably 10⁷. Doses of microspheres of less than 500 mg and preferably 1 - 100 mg are suitable. Harding *et al* ((1973) *J. Nucl. Med.* **14,** 579) have calculated that a 1 mg dose of albumin microspheres of 15 µm in diameter (equivalent to 5.7 x 10⁵ particles) will block only 0.14% of the pulmonary arterial circulation.

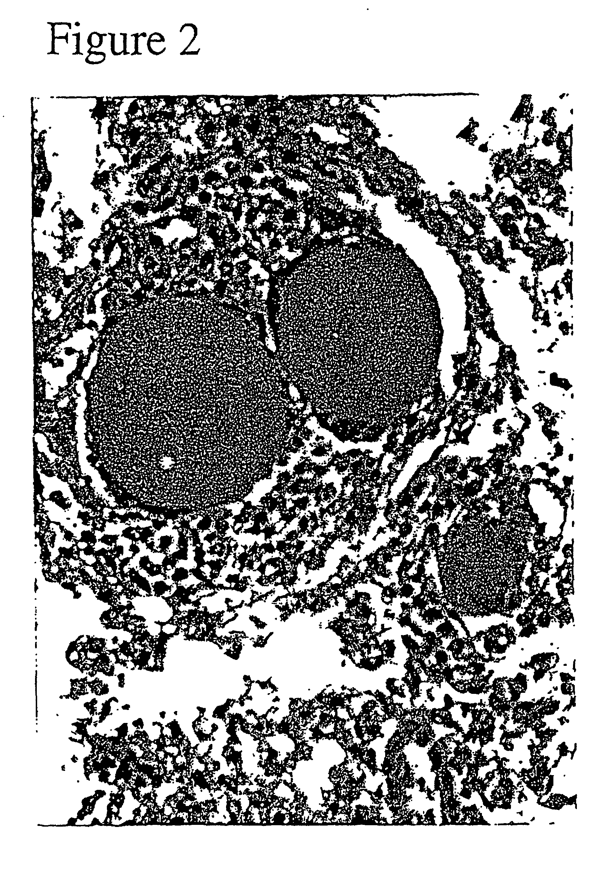

A photomicrograph showing how such microspheres can block the capillary beds to provide excellent contact between the particle surface and the endothelial cells of the lung capillary is shown in Figure 2. The long term residence of low doses of such microspheres in the pulmonary capillaries would appear to be without pathological effect (Davis and King, (1974) *J. Nucl. Med.* **15**, 436). The degraded microsphere, once it is too small to block the capillary, will continue within the circulatory system and be removed by the liver through the normal process of particle recognition and subsequent phagocytosis. Here, the microsphere and its contents will be taken up by the Kupffer cells of the liver into a lysosomal compartment leading to the further degradation of the microsphere and more particularly to the degradation of the surface attached DNA material. Thus, the new delivery system for the lung should be able to provide site specific expression of a gene in the lung since any DNA material finding its way to the liver is likely to be effectively degraded and unable to provide expression.

A composition of the invention may be administered intravenously or intra-arterially to a mammal. It may be delivered intravenously or intra-arterially to the lung endothelium of a mammal. It may also be delivered intra-arterially to a body compartment such as the joints, nasal cavity, vagina, rectum, eye, gastrointestinal tract, ear or buccal membrane. It may be administered such that the DNA is delivered to the joints, nasal cavity, vagina, rectum, eye, gastrointestinal tract, ear or buccal membrane. For example, the composition may be administered into a blood vessel that supplies blood to the desired compartment.

A further aspect of the invention is the use of a composition of the invention in the manufacture of a medicament for the treatment of a mammal in need of gene therapy.

A further aspect of the invention is a kit of parts for delivery of the compositions of the invention to a mammal, comprising compositions of the invention and sterile carrier components. For example, the pulmonary delivery system of this invention can be produced just prior to administration using sterile components or a preformed heterocoagulated particulate system can be produced using sterile components and then freeze dried for subsequent resuspension and administration.

The present invention will now be described, by way of example, in more detail with reference to the following figures and examples.
Figure 1: DNA-chitosan nanoparticle conjugates adsorbed to biodegradable microparticle
Figure 2: photomicrograph showing microspheres in the lung capillary bed
Figure 3: adsorption of DNA-chitosan complexes on negative charged starch microspheres. Approximately 50 µg DNA in 1.5 ml distilled water.
Figure 4: microspheres with and without adsorbed complex.

### Example 1: Preparation of DNA-chitosan conjugates, and their adsorption on negatively charged starch microspheres.

The following example describes the preparation of a starch microsphere particle carrying a CMV-CAT plasmid complexed with chitosan.

### A. Preparation of DNA-CTS (chitosan) complexes

26.4 mg of chitosan hydrochloride was dissolved in 18 ml of water. The solution was passed through a 0.2 µm membrane filter (CTS solution). 15 ml of CTS solution was added dropwise into 50 ml of DNA solution (containing 2.2 mg pCAT-DNA) under magnetic stirring until the solution just started to be turbid (about 2 ml). The remaining CTS solution was then added quickly into the suspension (ml by ml). The resulting suspension (containing 33.8 µg/ml of DNA and 338 µg/ml of CTS) was stored at 4°C until use. The particles had a size of about 200 nm and a surface charge (zeta potential) of +35 mV.

### B. Purification of DNA-CTS complexes

0.4 ml of DNA/CTS complex suspension was added to a filter-centrifugation tube (100 nm cutoff) (Amicon) and centrifuged at 6500 rpm for 10 min. The complexes were resuspended in 0.3 ml of water and collected. 15 ml of sample was treated and the resulting cleaned complexes were contained in about 10 ml of water.

### C. Quantitative methods for assay of DNA-CTS complexes (spectrophotometry method)

The turbidity of the complex suspension at the range 350-600 nm is too low to use for quantitative measurement. However, maximum absorbance at 259 nm (λₘₐₓ of DNA) can be used as an alternative parameter for measurement of the complex. A good linear relation between the UV absorbance at 260 nm and the concentration of DNA-CTS complexes in the suspension fraction range of 0.2 B 1.0 (approx. 10-50 µg/ml DNA) was obtained.

### D. Adsorption of the complexes onto starch microspheres

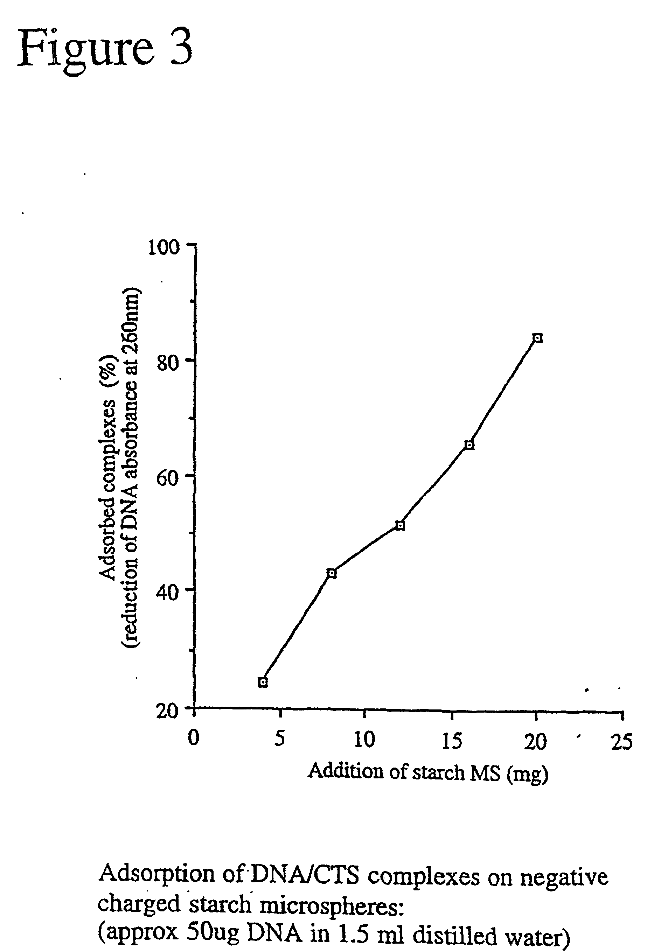

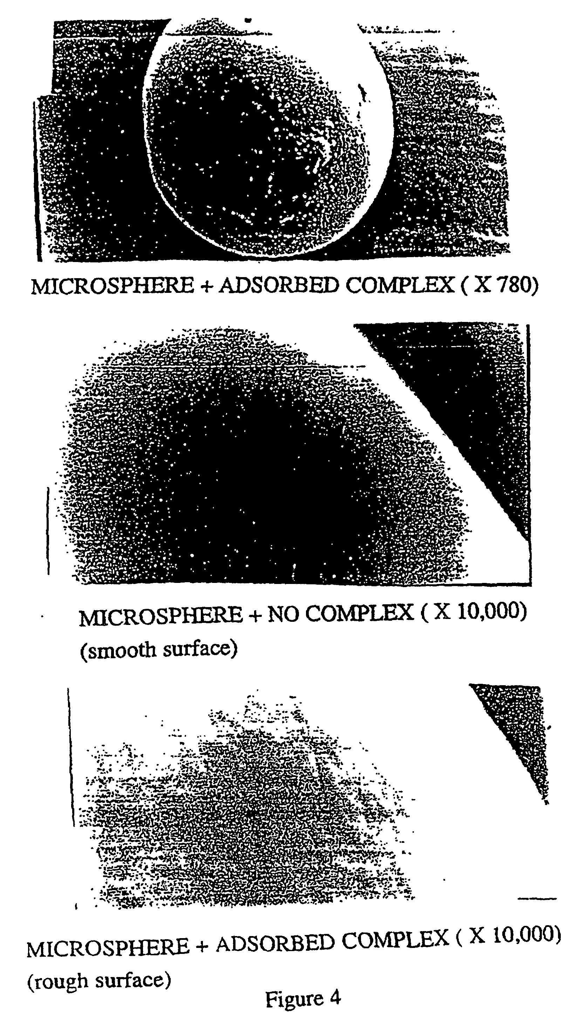

0.1 ml of formulation B and 0.5 ml of 4% (w/v) starch microsphere suspension comprising carboxylated microspheres of an approximate size 50 µm (Cadoxamer from Perstorp, Sweden) were added into 1.0 ml of DNA/CTS complex suspension. 0.5 ml of the starch microsphere suspension without the DNA-CTS complex was used as blank control and the 1.0 ml of DNA-CTS complex suspension without starch microspheres as standard control. The final volume of the samples was adjusted to 1.5 ml by adding a suitable amount of water. The samples were shaken on a vibratory shaker at room temperature for 2 hours followed by centrifuging at 6500 rpm for 3 min. Only slight influence of the supernatant from starch microspheres on DNA measurement at 260 nm was found. The supernatant was taken and measured at 260 nm. The adsorption of the complexes was calculated by comparing the reduction of UV absorbance of the samples with that of DNA-CTS complex standard control (Figure 3). A linear increase in the surface adsorption of the DNA-CTS complex was found. The addition of 20 mg of starch microspheres gave rise to greater than 80% adsorption of the complex. Electronmicrographs showing adsorbed DNA-CTS complex on starch microspheres are shown in Figure 4.

### Example 2: Administration of the composition prepared as described in Example 1 to rabbits.

The composition described in Example 1 was prepared and the microspheres carrying the DNA-conjugate separated by centrifugation and dispersed in sterile water for injection. New Zealand White female rabbits in a group of 3 were injected intravenously via the marginal ear vein with 10⁶ particles in 0.5 ml. The animals were sacrificed 3 hours later and the lungs removed together with liver and spleen. Histological examination showed that the microspheres were largely sequestered by the lungs. The microspheres were found to deposit deeply into the capillary beds of the lungs.

### Example 3: Assay of gene expression - CAT analysis

### Materials

The reporter gene pCAT-DNA driven by a CMV promoter was supplied by GeneMedicine, Houston, USA. ApCAT-DOTMA (N[1-(2,3-dioleoyloxy)-propyl]-NNN-trimethylammonium chloride) complex was used as a control material. This was prepared as outlined by Felgner *et al*, (1987), *Proc. Natl. Acad. Sci. (UA)* **84,** 7415-7417.

### Methods

A control pCAT DNA solution was prepared by the dilution of the pCAT-DNA stock solution (0.811 mg/ml) in autoclaved PBS. To 0.863 ml of the pCAT-DNA stock solution was added 13.137 ml of autoclaved PBS. This provided 14.0 ml of control pCAT-DNA solution (0.05 mg/ml), sufficient to dose six rabbits with 100 µg of pCAT-DNA in a volume of 2 ml. The solution was stored at 4°C and warmed to room temperature 15 min prior to dosing the rabbits.

A pCAT-DNA DOTMA formulation supplied in the form of lyophilised powder was reconstituted on the appropriate study day using sterile water. The resultant liquid formulations contained a sufficient concentration of plasmid DNA to administer a dose of 100 µg of pCAT-DNA in a volume of 2 ml. The formulation was stored at 4°C and warmed to room temperature 15 min prior to dosing the rabbits.

### CAT ELISA

The CAT ELISA procedure for the analysis of CAT in the animal tissues was set up following the protocol supplied by the manufacturer of the kit (Boerhinger Mannheim). The assay is based on the sandwich ELISA principle, where the anti-CAT antibody is prebound to the surface of the microtitre plate modules, the CAT enzyme in the extracts of the tissues, binds specifically to the immobilised anti-CAT antibody and digoxogenin-labeled antibody to CAT (anti-CAT-DIG) binds to CAT enzyme. The detection antibody, peroxidase conjugated anti-digoxigenin antibody (anti-DIG-POD) binds to digoxigenin. In the final step the peroxidase substrate (ABTS; 2,2'-azinobis[3-ethylbenzthiazoline sulphonic acid]) is catalysed by peroxidase yielding a colour reaction product which is enhanced by the use of a substrate enhancer. The absorbance of this colour is then directly correlated to the amount of CAT enzyme present in the standards and the extracts.

### Validation of CAT ELISA

Prior to analysing the test samples, the CAT ELISA procedure was validated. Since the appropriate quality control tissue samples containing CAT were not available, both the intra-day and inter-day variations were determined using the standard curves. These curves were prepared using the CAT standards supplied in the CAT ELISA kit at CAT concentrations of 0.0, 15.6, 31.25, 62.5, 125, 250 and 500 pg/ml. The intra-day variation was determined by preparing the standard curve in triplicate. Both for the infra-day and inter-day variation the coefficient of variation (%CV) at each standard concentration was calculated.

### Validation of the tissue extraction procedures

The tissue extraction procedure was initially established by taking the various tissue samples from two control rabbits dosed with "naked" plasmid DNA and storing separately at -80°C. 0.5 ml of ice-cold tissue extraction buffer (10 mM Tris pH 7 - 8; 150 mM NaCl, 1 mM EDTA, 0.5% Triton X- 100, 100 µM Leupeptin, 1.0 µM pepstatin and 0.25 mM PMSF) was added to the tube containing the tissue sample and homogenised four different times (0.6, 1.2 or 2.4 min) using a Mini-Bead Beater at 4°C. The homogenate was transferred to a sterilised microfuge tube (1.5 ml) and centrifuged in an MSE Microfuge at 13,000 rpm for 20 min at 4°C. The supernatant (ie. tissue extract) was transferred to a sterilised, microfuge tube (1.5 ml) and placed on ice. An aliquot of the tissue extract was diluted (1 in 20) with PBS and assayed for protein concentration using the BCA protein assay. Each sample was analysed in duplicate and corrected for dilution in order to obtain the total protein concentration (mg/ml) in the tissue extract.

The CAT ELISA procedure was validated for the analysis of CAT in the gastrointestinal tissue extracts. Firstly, the effect of protein concentration on the determination of CAT concentration in the various tissue extracts using the CAT ELISA procedure was assessed. Tissue samples from the control rabbits were extracted in ice-cold tissue extraction buffer (0.5 ml) as described above but were homogenised for 1.5 min (3 cycles of 30 sec. homogenisation and 30 sec. rest). After determining the protein concentration, the tissue extracts were diluted with Sample Buffer (CAT ELISA kit) to produce extract solutions with protein concentrations of approximately 0.5, 1.0 and 2.0 mg/ml). Each of these diluted extracts were spiked with CAT standard (CAT ELISA kit) to given final CAT concentrations of 25 and 100 pg/ml. The spiked samples were then analysed in duplicate for CAT concentration and the percentage recovery of CAT in each sample was calculated.

Secondly, the effect of extraction procedure on the stability of CAT in the various tissues was established. This was carried out by extracting the tissue samples from the above control rabbits with extraction buffer containing CAT concentrations of 50 or 200 pg/ml. After determining the protein concentrations each sample extract was diluted (1 in 2 or 1 in 4) with the Sample Buffer. Each diluted sample was analysed in duplicate for CAT concentration. After correcting for dilution the percentage recovery of CAT in each tissue extract was calculated.

### Example 4: Administration to a patient

A DNA: chitosan complex is prepared according to the method outlined in Example 1 above, with the exception that the DNA plasmid is a DNA plasmid expressing cystic fibrosis transmembrane conductance regulator.

The complex preparation is controlled to obtain particles in the range 100 to 300 nm. The particles are freeze dried after the addition of 2.5 to 5% mannitol.

Immediately prior to administration to a patient, an amount of DNA:chitosan complex equivalent to 100 µg DNA is dispersed in 1.0 ml water for injection (USP) together with 20 mg of starch microspheres (Cadoxomer, Perstorp, Sweden). The suspesnsion is injection into a vein of a patient.

The material is also available as a kit consisting of a mixture of the required amount of freeze dried starch microspheres and DNA:chitosan complex, a vial containing 1 ml water for injection and a syringe for injection.

## Claims

1. A composition comprising biodegradable microspheres wherein the microspheres carry a net negative charge and to which is adsorbed positively charged particles of a smaller size, wherein such positively charged particles comprise a conjugate of DNA and a cationic compacting agent.

2. A composition according to Claim 1 wherein the biodegradable microspheres are prepared from crosslinked soluble starch, albumin, gelatin, heparin, carboxydextran, dextran, dextran sulphate, alginate, polylactide or their admixtures.

3. A composition according to Claim 1 or Claim 2 wherein the cationic compacting agent is chitosan.

4. A composition according to any one of Claims 1-3 wherein the biodegradable microspheres have an average size larger than 5 µm.

5. A composition according to any one of Claims 1-4 wherein the biodegradable microspheres carry a zeta potential of at least -5 mV before adsorption of the DNA conjugate.

6. A composition according to any one of Claims 1-5 wherein the DNA conjugate particles have an average size between 10 and 500 nm.

7. A composition according to any one of Claims 1-6 wherein the DNA conjugate has a positive surface charge (zeta potential) from 5 to 100 mV.

8. A method of preparing a composition which comprises 1) preparing positively charged particles comprising a conjugate of DNA and a cationic compacting agent, and 2) adsorbing of said positively charged particles on to biodegradable microspheres carrying a net negative charge.

9. A composition obtainable by a method according to Claim 8.

10. A composition according to any one of Claims 1-7 or 9, wherein the microspheres act as carriers for the delivery of DNA to the endothelial cells of a vascular bed.

11. A composition according to any one of Claims 1-7, 9 or 10 for use in medicine.

12. A composition according to Claim 11 wherein the dose of administered microspheres is less than 10 x 10¹⁰ in number or of a quantity less than 500 mg.

13. Use of a composition according to any one of Claims 1-7, 9 or 10 in the manufacture of a medicament for a method of delivery of DNA to a mammal wherein said composition is to be administered intravenously or intra-arterially to a mammal.

14. Use according to Claim 13 in which the composition is to be delivered intravenously or intra-arterially to the lung endothelium of a mammal.

15. Use of a composition according to any one of Claims 1-7, 9 or 10 in the manufacture of a medicament for a method of treatment wherein said composition is to be delivered to the joints, nasal cavity, vagina, rectum, eye, gastrointestinal tract, ear or buccal membrane.

16. Use of a composition according to any one of Claims 1-7, 9 or 10 in the manufacture of a medicament for a method of treatment wherein said composition is to be administered such that the DNA is delivered to the joints, nasal cavity, vagina, rectum, eye, gastrointestinal tract, ear or buccal membrane.

17. Use according to Claim 13 in which the DNA is to be delivered to the lung endothelium of a mammal.

18. Use of a composition according to any one of Claims 1-7, 9 or 10 in the manufacture of a medicament for the treatment of a mammal in need of gene therapy.

19. A kit of parts for delivery of a composition according to any one of Claims 1-7, 9 or 10 to a mammal, comprising said composition and sterile carrier components.

20. A kit of parts for delivery of a composition according to any one of Claims 1-7, 9 or 10 to a mammal, comprising biodegradable microspheres carrying a net negative charge; positively charged particles of a smaller size, wherein such positively charged particles comprise a conjugate of DNA and a cationic compacting agent; and sterile carrier components.

## Patentansprüche

1. Zusammensetzung, die biologisch abbaubare Mikrokügelchen umfaßt, wobei die Mikrokügelchen eine negative Nettoladung tragen, an die positiv geladene Teilchen mit kleinerer Größe adsorbiert sind, wobei diese positiv geladenen Teilchen ein DNA-Konjugat und ein kationisches Verdichtungsmittel umfassen.

2. Zusammensetzung nach Anspruch 1, bei der die biologisch abbaubaren Mikrokügelchen aus quervemetzter löslicher Stärke, Albumin, Gelatine, Heparin, Karboxydextran, Dextran, Dextransulfat, Alginat, Polyactid oder ihren Mischungen hergestellt sind.

3. Zusammensetzung nach Anspruch 1 oder 2, bei der das kationische Verdichtungsmittel Chitosan ist

4. Zusammensetzung nach einem der Ansprüche 1 bis 3, bei der die biologisch abbaubaren Mikrokügelchen eine mittlere Größe von mehr als 5 µm besitzen.

5. Zusammensetzung nach einem der Ansprüche 1 bis 4, bei der die biologisch abbaubaren Mikrokügelchen vor der Adsobtion des DNA-Konjugats ein Zetapotential von wenigstens - 5mV tragen.

6. Zusammensetzung nach einem der Ansprüche 1 bis 5, bei der die DNA-Konjugat-Teilchen eine mittlere Größe zwischen 10 und 500 nm besitzen.

7. Zusammensetzung nach einem der Ansprüche 1 bis 6, bei der das DNA-Konjugat eine positive Oberflächenladung (Zetapotential) von 5 bis 100 mV besitzt.

8. Verfahren zur Zubereitung einer Zusammensetzung, das folgende Schritte umfaßt 1) Zubereitung von positiv geladenen Teilchen, die ein DNA-Konjugat und ein kationisches Verdichtungsmittel umfassen, und 2) Adsorbieren dieser positiven geladenen Teilchen an biologisch abbaubare Mikrokügelchen, die eine negative Nettoladung tragen.

9. Zusammensetzung, die durch ein Verfahren gemäß Anspruch 8 erhalten werden kann.

10. Zusammensetzung nach einem der Ansprüche 1 bis 7 oder 9, bei der die Mikrokügelchen als Träger für die Abgabe von DNA an die endothelialen Zellen eines vaskulären Bettes dienen.

11. Zusammensetzung nach einem der Ansprüche 1 bis 7, 9 oder 10 zur Verwendung im medizinischen Bereich.

12. Zusammensetzung nach Anspruch 11, bei der die Dosis der verabreichten Mikrokügelchen weniger als 10 x 10¹⁰ in Bezug auf die Anzahl oder eine Menge von weniger als 500 mg beträgt.

13. Verwendung von Zusammensetzungen nach einem der Ansprüche 1 bis 7, 9 oder 10 bei der Herstellung eines Medikamentes für ein Verfahren zur Abgabe von DNA an ein Säugetier oder einen Menschen, wobei besagte Zusammensetzung intravenös oder intraarteriell einem Säugetier oder einem Menschen verabreicht werden soll.

14. Verwendung nach Anspruch 13, bei der die Verwendung intravenös oder intraarteriell dem Lungenendothelium eines Säugetiers oder eines Menschen zugeführt werden soll.

15. Verwendung einer Zusammensetzung nach einem der Ansprüche 1 bis 7, 9 oder 10 bei der Herstellung eines Medikamentes für ein Behandlungsverfahren, bei dem diese Zusammensetzung den Gelenken, dem Nasenhohlraum, der Vagina, dem Rektum, dem Auge, dem Verdauungstrakt, dem Ohr oder der Rachenmembran zugeführt werden soll.

16. Verwendung einer Zusammensetzung nach einem der Ansprüche 1 bis 7, 9 oder 10 bei der Herstellung eines Medikamentes für ein Behandlungsverfahren, bei dem die Zusammensetzung so verabreicht werden soll, daß die DNA den Gelenken, dem Nasenhohlraum, der Vagina, dem Rektum, dem Auge, dem Verdauungstrakt, dem Ohr oder der Rachenmembran zugeführt wird.

17. Verwendung nach Anspruch 13, bei der die DNA dem Lungenendothelium eines Säugetiers oder Menschen zugeführt werden soll.

18. Verwendung einer Zusammensetzung nach einem der Ansprüche 1 bis 7, 9 oder 10 bei der Herstellung eines Medikamentes für die Behandlung eines Säugetiers oder eines Menschen, das bzw. der einer Gentherapie bedarf.

19. Teilesatz für die Verabreichung einer Zusammensetzung nach einem der Ansprüche 1 bis 7, 9 oder 10 an ein Säugetier oder einen Menschen, wobei der Teilesatz besagte Zusammensetzung und sterile Trägerkomponenten umfaßt.

20. Teilesatz für die Abgabe einer Zusammensetzung nach einem der Ansprüche 1 bis 7, 9 oder 10 an ein Säugetier oder einen Menschen, wobei der Teilesatz folgendes umfaßt: biologisch abbaubare Mikrokügelchen, die eine negative Nettoladung tragen, positiv geladene Teilchen kleinerer Größe, wobei diese positiv geladenen Teilchen ein DNA-Konjugat und ein kationisches Verdichtungsmittel umfassen, sowie sterile Trägerkomponenten.

## Revendications

1. Composition comprenant des microsphères biodégradables, dans laquelle les microsphères transportent une charge nette négative et auxquelles sont adsorbées des particules chargées positivement de taille inférieure, dans laquelle ces particules chargées positivement comprennent un conjugué d'ADN et un agent de compression cationique.

2. Composition selon la revendication 1, dans laquelle les microsphères biodégradables sont préparées à partir d'amidon, d'albumine, de gélatine, d'héparine, de carboxy dextrane, de dextrane, de sulfate de dextrane, d'alginate, de polylactide réticulés ou de leurs mélanges.

3. Composition selon la revendication 1 ou la revendication 2, dans laquelle l'agent de compression cationique est du chitosan.

4. Composition selon l'une quelconque des revendications 1 à 3, dans laquelle les microsphères biodégradables ont une taille moyenne supérieure à 5 µm.

5. Composition selon l'une quelconque des revendications 1 à 4, dans laquelle les microsphères biodégradables transportent un potentiel zêta d'au moins -5 mV avant l'adsorption du conjugué d'ADN.

6. Composition selon l'une quelconque des revendications 1 à 5, dans laquelle les particules du conjugué d'ADN ont une taille moyenne comprise entre 10 et 500 nm.

7. Composition selon l'une quelconque des revendications 1 à 6, dans laquelle le conjugué d'ADN présente une charge superficielle positive (potentiel zêta) allant de 5 à 100 mV.

8. Procédé de préparation d'une composition qui comprend les étapes consistant à 1) préparer des particules chargées positivement comprenant un conjugué d'ADN et un agent de compression cationique, et 2) à adsorber lesdites particules chargées positivement sur des microsphères biodégradables transportant une charge nette négative.

9. Composition pouvant être obtenue grâce à un procédé selon la revendication 8.

10. Composition selon l'une quelconque des revendications 1 à 7 ou 9, dans laquelle les microsphères agissent comme véhicules destinés à délivrer de l'ADN aux cellules endothéliales d'un lit vasculaire.

11. Composition selon l'une quelconque des revendications 1 à 7, 9 ou 10 destinée à être utilisée en médecine.

12. Composition selon la revendication 11, dans laquelle la dose de microsphères administrées est inférieur au nombre de 10 x 10¹⁰ ou de quantité inférieure à 500 mg.

13. Utilisation d'une composition selon l'une quelconque des revendications 1 à 7, 9 ou 10 dans la fabrication d'un médicament pour un procédé de délivrance d'ADN à un mammifère, dans laquelle ladite composition doit être administrée par voie intraveineuse ou intra-artérielle à un mammifère.

14. Utilisation selon la revendication 13, dans laquelle la composition doit être administrée par voie intraveineuse ou intra-artérielle dans l'endothélium pulmonaire d'un mammifère.

15. Utilisation d'une composition selon l'une quelconque des revendications 1 à 7, 9 ou 10 dans la fabrication d'un médicament pour un procédé de traitement dans lequel ladite composition doit être délivrée dans les articulations, la cavité nasale, le vagin, le rectum, l'oeil, le tube digestif, l'oreille ou une membrane buccale.

16. Utilisation d'une composition selon l'une quelconque des revendications 1 à 7, 9 ou 10 dans la fabrication d'un médicament pour un procédé de traitement dans lequel ladite composition doit être administrée de telle sorte que l'ADN soit délivré dans les articulations, la cavité nasale, le vagin, le rectum, l'oeil, le tube digestif, l'oreille ou une membrane buccale.

17. utilisation selon la revendication 13, dans laquelle l'ADN doit être délivré dans l'endothélium pulmonaire d'un mammifère.

18. Utilisation d'une composition selon l'une quelconque des revendications 1 à 7, 9 ou 10 dans la fabrication d'un médicament pour le traitement d'un mammifère si une thérapie génique s'avère nécessaire.

19. Trousse de pièces destinée à la délivrance d'une composition selon l'une quelconque des revendications 1 à 7, 9 ou 10 à un mammifère, comprenant ladite composition et des composants véhicules stériles.

20. Trousse d'éléments destinée à la délivrance d'une composition selon l'une quelconque des revendications 1 à 7, 9 ou 10 à un mammifère, comprenant des microsphères biodégradables transportant une charge nette négative, des particules chargées positivement de taille inférieure, où ces particules chargées positivement comprennent un conjugué d'ADN et un agent de compression cationique, et des composants véhicules stériles.
